# EUROPEAN PATENT SPECIFICATION

(11) **EP 2 598 040 B1**
(45) Date of publication and mention of the grant of the patent: **02.09.2020**
(21) Application number: 11813034.3
(22) Date of filing: 26.07.2011
(51) Int. Cl.: A61B 10/02, A61B 10/00, A61B 90/00

(54) **FULL CORE BIOPSY DEVICE**
VOLLSCHNITT-BIOPSIEVORRICHTUNG
DISPOSITIF DE BIOPSIE À NOYAU PLEIN

(30) Priority: 27.07.2010 US 368119 P
(43) Date of publication of application: 05.06.2013
(73) Proprietor: Pave, LLC, Indianapolis, IN 46240 (US)
(72) Inventor: SUNDHEIMER, Herbert, Alan, Franklin, IN 46131 (US); IRELAND, Dan, C., Martinsville, IN 46151 (US); MILLER, Sean, Greenwood, IN 46143 (US)
(74) Representative: Hering, Hartmut
(86) International application number: PCT/US2011/045322
(87) International publication number: WO 2012/015801

(56) References cited:
- DE-A1- 10 107 513
- JP-A- 2005 137 454
- US-A- 4 512 344
- US-A- 4 907 598
- US-A- 5 490 521
- US-A- 5 535 755
- US-A- 5 615 690
- US-A- 5 655 542
- US-A- 6 083 237
- US-A1- 2007 106 176
- US-A1- 2008 234 602
- US-A1- 2009 204 020

## Description

### Background

The present invention relates to devices for obtaining a tissue biopsy sample, and more particularly to a device for obtaining a soft tissue core biopsy sample.

Clinicians obtain biopsy specimens for the purpose of diagnosing, staging and grading disease states. One type of biopsy device is a core biopsy needle, which typically operates by coaxial action of an inner needle or stylet having a specimen notch and an outer needle or cannula having a sharp end, with the tip of the inner stylet proud of the end of the outer cannula. The stylet is advanced so that the specimen notch is exposed to tissue, which prolapses into the notch. The cannula is then advanced over the stylet so that the sharp end of the cannula severs the tissue leaving the specimen in trapped within the notch. The volume of the specimen is limited by the notch and the inner diameter of the cannula.

In many biopsy situations, clinicians may desire a full, rounded core sample. The larger cross-section and volume of tissue can provide a more accurate assessment of the tissue pathology. It is also desirable to obtain full, clean core samples that have not been crushed by devices penetrating into tissue, since "crush artifacts" can compromise the analysis of the retrieved sample. In addition, the larger volume of the full core may often provide enough tissue so that only a single pass of the biopsy needle is required. Moreover, it may be desirable to obtain a core sample without having to penetrate past a desired depth of tissue in order to obtain a corresponding desired depth of core sample. It is preferable to insert a biopsy needle only as far as necessary to obtain the desired core sample.

Coring devices are well known for obtaining samples of hard tissue such as bone. These coring devices include an outer needle having a sharpened edge that is manually pushed and rotated into bone. In this case the rigidity of the tissue - e.g., bone - assures a generally intact full core sample. However, soft tissues do not have the same rigidity and are prone to flow away from the cutting needle. One known device capable of obtaining full core samples of soft tissues is the FNA (fine needle aspiration) device. This device typically includes an outer needle having a sharpened edge at the tip. The outer needle is typically manually moved back and forth while rotating the needle into the target tissue. The use of aspiration helps pull the tissue into the FNA needle. Obtaining core samples in this manner requires a certain amount of dexterity to "tease" the tissue into the needle. Thus, the success of the biopsy is typically technique dependent. Moreover, the FNA procedure takes time ensure that a suitable sample is obtained. In many cases, multiple attempts are required to obtain an adequate sample, which exposes the patient to further discomfort and pain.

Partially and fully automated biopsy devices simplify and shorten the biopsy procedure, but generally eliminate the ability to "tease" the tissue into the coring cannula or needle. While an FNA device may be advanced into soft tissue at a rate of 2cm/min, a typical automated biopsy device advances the outer needle into the tissue at a rate of 200 cm/min. At these speeds the coring needle has a tendency to push the tissue aside, rather than to draw the tissue into the needle. There is a continuing need for biopsy devices, and particularly full core biopsy devices, which can quickly and efficiently obtain large, intact tissue samples. The need is particularly acute for soft tissue samples because the soft tissue can be difficult to extract and retain without damage to the tissue. A biopsy device is disclosed in JP 2005 137454 A.

### Summary

According to the invention, a biopsy device is disclosed comprising: an outer needle hub; an outer needle defining a longitudinal axis along a length thereof and coupled at a proximal end to the outer needle hub, the outer needle including: an outer needle tip at an opposite distal end; a tissue slicing feature defined at said tip configured for cutting tissue; and an inner surface including a countersink formed at said tip, said countersink tapered from a distal most end of said outer needle tip; an inner needle hub; an inner needle coupled at a proximal end to said inner needle hub, the inner needle including an inner needle tip at an opposite distal end, said inner needle coaxially disposed within said outer needle, wherein said countersink is tapered at approximately a one to two degree angle relative to the inner surface. In certain embodiments, the tissue retention feature may include a groove defined in the inner surface.

In certain embodiments, a biopsy device comprises coaxially disposed outer and inner needles and a mechanism coupled to the needles for charging and firing the outer needle relative to the inner needle to capture a tissue sample within the outer needle. The inner needle and the outer needle are sized so that the inner needle tip is always proximal of a distalmost end of the outer needle tip when the outer needle is charged and fired. In certain embodiments, the outer needle includes a tissue slicing feature having a plurality of prongs and valleys therebetween, with the valleys defining a base diameter. In one feature, the prongs of the tissue slicing feature have a height and the inner needle and the outer needle are sized so that the inner needle tip is initially positioned, before the outer needle is fired, at a distance from the valleys approximately equal to one quarter of the height.

The present disclosure further provides a biopsy device having coaxially disposed inner and outer needles and a vacuum generating component. The vacuum generating component includes a chamber in communication with the proximal end of the inner needle, a piston coupled to the proximal end of the inner needle and slidably disposed within the chamber, and a handle coupled to the proximal end of the inner needle configured to manually pull the inner needle and the piston therewith to draw a vacuum within the chamber.

A method of obtaining a tissue sample is using a biopsy device is provided that comprises introducing the biopsy device into a tissue site where a tissue sample is to be obtained, obtaining the tissue sample with the outer needle and selectively drawing a vacuum in the inner needle to retain the tissue sample within the outer needle as the device is extracted from the tissue site. In certain embodiments, the vacuum is drawn only as the biopsy device is extracted from the tissue site.

An outer needle is provided for use in a biopsy device having an inner needle to be slidably disposed within the outer needle and a charging and firing mechanism coupled to the inner and outer needles to advance the outer needle over the inner needle to obtain a tissue sample. In certain embodiments, the outer needle comprises a proximal end to be coupled to the charging and firing mechanism, an outer needle tip at an opposite distal end, an outer surface having a tissue slicing feature at the tip configured for cutting tissue, and an inner surface including a countersink formed at the tip. The outer needle may further include an additional tissue retention feature defined in the inner surface between the tissue slicing feature and the proximal end of the outer needle, such as a spiral groove defined in the inner surface.

The above described features and advantages, as well as others, will become more readily apparent to those of ordinary skill in the art by reference to the following detailed description and accompanying drawings. While it would be desirable to provide a biopsy device that includes one or more of these advantageous features, the teachings disclosed herein extend to those embodiments which fall within the scope of the appended claims, regardless of whether they accomplish one or more of the above-mentioned advantages.

### Description of the Figures

**FIG. 1** is a top cross-sectional view of a full core biopsy device of the prior art.
**FIG. 2** is a side view of an outer needle component of the full core biopsy device shown in **FIG. 1****.**
**FIG. 3** is a side view of an inner needle component of the full core biopsy device shown in **FIG. 1****.**
**FIG. 4** is an enlarged side view of the end of an outer needle component for use with the full core biopsy device shown in **FIG. 1****,** with the inner needle in its extended position.
**FIG. 5** is an enlarged side view of the end of the outer needle component for use with the full core biopsy device shown in **FIG. 1****,** with the inner needle in its retracted position.
**FIG. 6** is an enlarged view of an outer needle according to a further embodiment for use with the full core biopsy core device of **FIG. 1****.**
**FIG. 7** is a side cross-sectional view of the outer needle component shown in **FIG. 6****.**
**FIG. 8** is an enlarged end perspective view of an outer needle and an inner needle with a filter element for use with some embodiments of the full core biopsy core device of **FIG. 1****.**
**FIG. 9** is a perspective view of a full core biopsy device with a vacuum element according to one aspect of the present disclosure.
**FIG. 10** is a side partial cross-sectional view of an inner needle component having a vent opening at one end for use with the full core biopsy device shown in **FIG. 1****.**
**FIG. 11** is a perspective view of an automated biopsy device capable of utilizing the full core biopsy components disclosed herein.
**FIG. 12** is a side view of a flexible needle biopsy device capable of utilizing the full core biopsy components disclosed herein.
**FIG. 13** is an enlarged side view of an outer needle and stylet of a full core biopsy device disclosed herein.
**FIG. 14** is an enlarged side view of an outer needle and stylet of a further embodiment of a full core biopsy device disclosed herein.

### Detailed Description

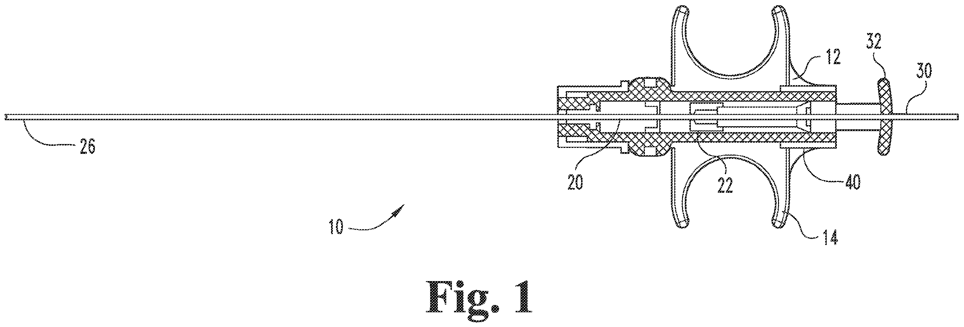

One type of core biopsy device **10** is shown in **FIG. 1****.** The device may include features found in the SABD™ core biopsy system sold by US Biopsy of Franklin, Indiana, or similar devices capable of obtaining a core tissue sample from a patient. Although the present disclosure relates to a core biopsy device, the features disclosed herein may be incorporated into other types of tissue sampling or tissue biopsy devices. The device **10** includes a housing **12** that defines finger handles **14** to be grasped by the clinician during a biopsy procedure. The device can include an outer cannula or needle **20** and an inner stylet, cannula or needle **30** coaxially extending through the outer needle **20**.

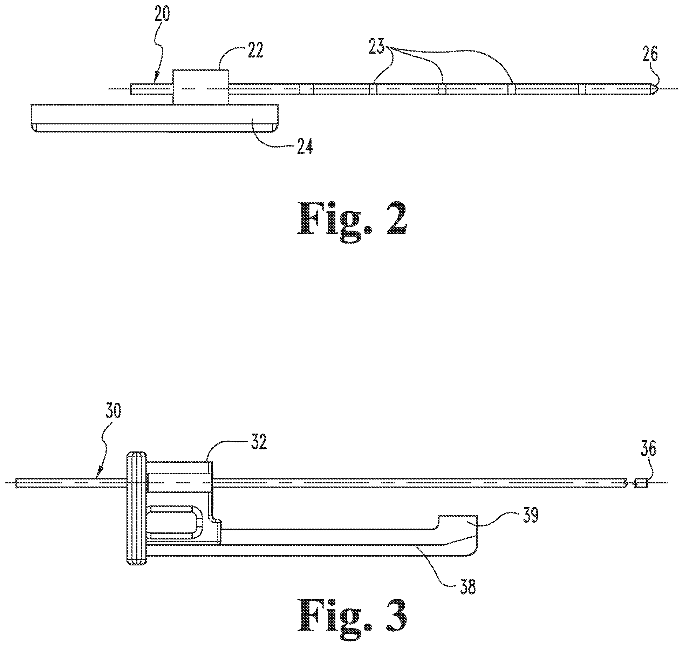

The biopsy device **10** incorporates a mechanism for charging and firing the outer needle relative to the inner needle in order to capture a tissue sample. One embodiment of a firing mechanism is described herein although other mechanisms are contemplated that permit charging and firing the outer needle relative to the inner needle to obtain a tissue sample, including semi or fully automated systems. As shown in more detail in **FIG. 2****,** the outer needle **20** is fixed within an outer needle hub **22** mounted on an outer needle carriage **24.** Similarly, as shown in **FIG. 3****,** the inner needle **30** is fixed within an inner needle hub **32** mounted on an inner needle carriage **38.** The inner needle carriage **38** includes a tab **39** for engaging the outer needle carriage **24** when the biopsy device **10** is charged. The outer needle **20** may include markings **23** used to determine the depth of the outer needle **20** upon insertion into the patient.

Referring back to **FIG. 1****,** the device **10** includes a spring **40** disposed between the housing **12** and the outer needle hub **22.** As is known, the device **10** may include a latch (not shown) that holds the outer needle **20** in its charged position. As with many similar biopsy devices, the device **10** is charged by pulling back on the inner needle hub **32,** which in turn pulls the outer needle carriage **24** back until it is engaged by the latch. As the outer needle hub **22** is retracted it compresses the spring **40** within the housing **12.**

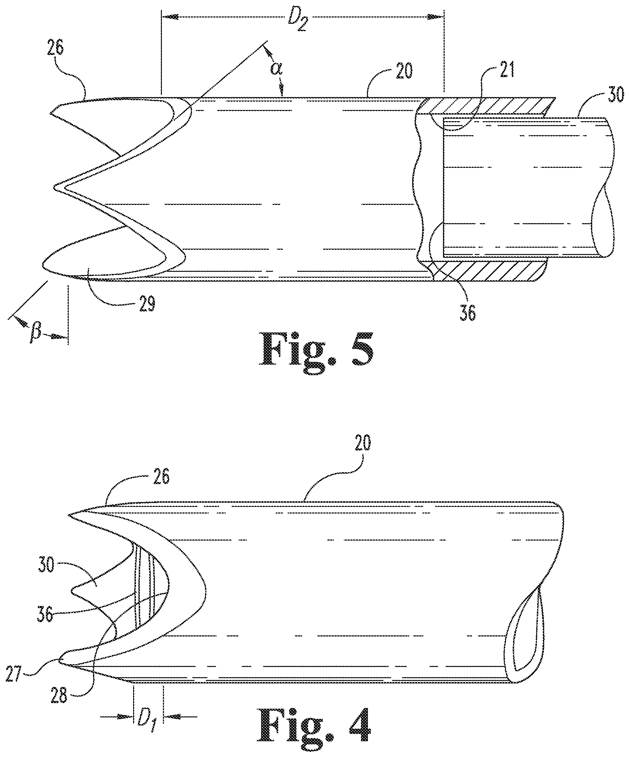

The biopsy device **10** may be fired by pushing the inner needle hub **32** forward so that the tab **39** trips the latch, although other firing mechanisms may be implemented. Once the latch is released the spring **40** propels the outer needle **20** forward over the inner needle and into the subject tissue. In a biopsy procedure, the clinician positions the tip **26** of the outer needle **20** against the tissue to be sampled, with the device in its charged position. When the device is fired, the outer needle **20** advances directly into the tissue so that a core of tissue is captured within the lumen **21** (**FIG. 5**) of the outer needle **20**. The device **10** can be removed from the patient and the tissue core retrieved from the outer needle **20** in a known manner.

As thus far described, the device **10** may be similar in structure and operation to the SABD™ biopsy system and other similar coaxial single action core biopsy devices. The present invention provides improvements to devices of this type and more particularly improvements to the outer and inner needles for use with such devices. However, it is understood that the features described herein may be incorporated into other types of tissue sampling or biopsy devices, including devices using a motor to cock and/or fire the device.

According to one aspect, the outer needle **20** is provided with a Franseen tip **26,** as shown in **FIGS. 4-5****.** The Franseen tip includes three or more prongs **27** separated by valleys **28** around the circumference of the outer needle tip **26.** In one specific embodiment, three prongs **27** are each defined at an angle α, which may be about 18°. The edge surfaces **29** of the prongs **27** may be defined at an angle β, which may be about 30°, to form a sharp cutting edge within the valleys **28.** The prongs **27** permits smooth piercing of the soft tissue as the outer needle **20** initially advances into the tissue and solid purchase once the outer needle has been fully advanced. The prongs **27** are configured to advance through the tissue without substantially compressing the tissue. The angled edge surfaces **29** of the prongs **27** act as guillotine cutters to slice cleanly through the tissue as the outer needle **20** advances.

In accordance with one aspect of the disclosed embodiments, the inner needle **30** is maintained in a retracted position relative to the tip **26** of the outer needle **20** when the device **10** is charged as well as when the device **10** is fired. Thus, as shown in **FIG. 4****,** the tip **36** of the inner needle **30** extends only a dimension **D1** from the base of the valleys **28** of the tip **26** when the inner needle hub **32** has been moved to trip the latch and release the outer needle **20** as explained above. When the device **10** is initially charged the inner needle tip **36** preferably does not extend beyond, or extends only minimally beyond, the base of the valleys **28** of the tip **26** of the outer needle **20**. Put another way, the tip **36** of the inner needle **30** is always offset rearward from the distalmost ends of the prongs **27** of the tip **26** of the outer needle **20**, as depicted in **FIG. 4****.** In one embodiment, the dimension **D1** is less than about one-fourth of the length of the prongs **27** (i.e., the distance between the base of the valleys **28** and the distal end or top of the prongs **27**).

It can be appreciated that in the charged position shown in **FIG. 4****,** the inner needle hub **32** is in position to fire the device **10.** Since the device **10** is fired by moving the inner needle hub **32** forward, as explained above, the tip **36** of the inner needle **30** may contact soft tissue if it resides too proud of the outer needle **20**. In prior devices the inner stylet extends beyond the end of the outer cutting cannula prior to firing which tends to push the soft tissue away from the cutting cannula, resulting in less than a full core sample or a sample with a crush artifact. In the embodiments disclosed herein, the arrangement of the inner needle **30** relative to the outer needle **20** in the charged and firing positions avoids this condition found in prior devices. It can be appreciated that this positional relationship is produced by appropriate sizing of the length of the outer needle **20** and inner needle **30** taking into account the configuration of the charging and firing mechanism. The inner needle **30** thus has a length that maintains the inner needle tip **36** in the position shown in **FIG. 4** when the inner needle hub **32** has been advanced to release the latch holding the outer needle hub **22** against the compressed firing spring **40**.

In yet another approach, the inner needle **30** can be mounted within the inner needle hub **32** to permit deliberate retraction of the inner needle **30** prior to firing to ensure that the inner needle tip **36** is clear of the outer needle tip **26.** Thus, a threaded arrangement may be incorporated between the inner needle **30** and the inner needle hub **32** configured so that rotation of the inner needle **30** backs the needle out from the inner needle hub **32.** As the inner needle **30** backs out relative to the inner needle hub **32,** the inner needle tip **36** is retracted from the outer needle tip **26.** The threaded engagement may be configured to prevent complete disengagement of the inner needle **30** from the inner needle hub **32** and may preferably incorporate a locking mechanism to lock the inner needle **30** in its retracted position when the biopsy device **10** is fired. With this embodiment, once the biopsy device **10** is charged the clinician takes the additional step of rotating the inner needle **30** to retract the tip **36** prior to firing the device. The inner needle **30** may be provided with a finger tab at its proximal end to facilitate manual rotation of the needle.

As shown in **FIG. 5****,** after the device **10** is fired, the inner needle **30** is offset rearward from the tip **26** of the outer needle **20** by a dimension **D2** because the outer needle **20** has been driven forward by the firing spring **40**. This dimension is calibrated to the length of the tissue core desired and is generally based on the throw of the device **10** achieved by the charging and firing mechanism - i.e., the distance that the outer needle **20** travels when propelled by the spring **40**. In certain full core biopsy devices the throw of the outer needle may be fixed, while in other such devices the throw may be adjustable to vary the length of the tissue sample that is obtained.

After the device has been fired, the excised tissue sample is retained within the end of the outer needle **20**. The inner needle **30** may then be used to expel the tissue sample. This can be accomplished by charging the device - i.e., by pulling back on the inner needle hub **32** - which withdraws the outer needle **20** to its initial charged position. With the outer needle **20** charged, the inner needle **30** can be freely advanced forward far enough to push the tissue sample out of the outer needle **20**, but not so far as to release the latch and dry fire the device **10.** The inner needle **30** would thus be advanced to the position shown in **FIG. 4****.** Since the inner needle **30** is used to expel the sample, it is desirable that the tip **36** of the inner needle **30** be immediately proximate the base of the valleys **28** of the tip **26** of the outer needle **20**. This position of the inner needle tip **36** will ensure that the soft tissue sample is dislodged from the outer needle **20** either freely or with only minor urging so as not to destroy the sample.

In certain uses of the device **10** the preferred initial step may be to insert an introducer and stylet to the biopsy site. The stylet is removed and the device **10** is charged and passed through the introducer until the outer needle tip **26** is initially engaged with the soft tissue. The device **10** is then fired and removed through the introducer. To remove the biopsy sample, the device **10** is charged again and the inner needle **30** is slowly advanced forward as the device **10** itself is moved backward over the receiving surface (similar to putting icing on a cake). Once the inner needle **30** reaches the end of its stroke, the biopsy sample should be fully and cleanly dislodged from the outer needle **20**.

It can be appreciated that the action of the inner needle **30** is an important factor in producing an intact full-core biopsy sample. The inner needle tip **36** may be closed so that tissue cannot migrate into the inner needle **30**. The inner needle tip **36** may be slightly concave to urge the trailing tissue toward the center of the inner needle **30**. The inner needle **30** is sized for a close running fit within the inner lumen **21** of the outer needle **20 (****FIG. 5****),** and to prevent passage of tissue into the gap between the inner needle **30** and outer needle **20**.

It can be appreciated that the combination of the Franseen tip **26** and the relative positioning between the inner needle **30** and outer needle **20** described above provides a significantly greater chance of obtaining a full, clean core biopsy sample that has not been crushed without having to penetrate past a desired depth of tissue in order to obtain a corresponding desired depth of core sample. The Franseen tip **26** of the device **10** provides a cleaner cut with only linear motion and without rotation of the outer needle **20**. This helps reduce the chance of crushing the sample. The relative position of the inner needle **30** and the outer needle **20** also reduces the chance of crushing the sample and helps reduce the depth in the tissue that the device **10** must travel to obtain its full, clean core biopsy sample.

As explained above, hard tissue, such as bone, is readily drawn into a biopsy device and withdrawn by manipulating the device. It is believed that the friction between the tissue core and the inner wall of the biopsy needle holds the core within the needle as it is withdrawn. In the case of hard tissue, the device can be manipulated slightly after the sample has been obtained to ensure that the sample cleanly separates from the native tissue. However, for soft tissue biopsies the ability to retain a long core sample and separate it from the native tissue has been problematic. It is believed that at least part of the difficulty is that there is insufficient friction force between the core sample and the inner wall of the biopsy needle to hold the core in place as the needle is withdrawn. The inherent resilience of the tissue often renders manipulation of the needle ineffective at separating the sample from the native tissue. It has been found that a particular aspect ratio of sample length to sample diameter is needed to obtain a good soft tissue sample. This aspect ratio varies depending upon the type of tissue and its lubricity.

For example, liver tissue is particularly lubricious so that the coefficient of friction between liver tissue and the smooth inner wall of a stainless steel biopsy needle is low. Where the coefficient of friction is low a longer sample is required to achieve the necessary friction force to resist the resistance of the tissue to tearing or separation. In the case of liver tissue, an aspect ratio of 20:1 - 25:1 has been found suitable for cleanly extracting a liver tissue sample. Thus, in the case of a 16 gauge needle having a nominal inner diameter of 0.0535in. the tissue sample must be about 1.1-1.3in. long in order to obtain a viable sample. A smaller 20 gauge needle requires a sample length of about 0.6-0.75in. to obtain a viable sample. To obtain a sample of these lengths the stroke of the outer needle **20** must exceed the desired sample length. Thus, to obtain a 1.3in. sample with a 16 gauge needle, the needle stroke is preferably at least 1.4 in. It has been found that for a prior full core biopsy needle construction viable tissue samples can sometimes be obtained but more often than not only after multiple attempts.

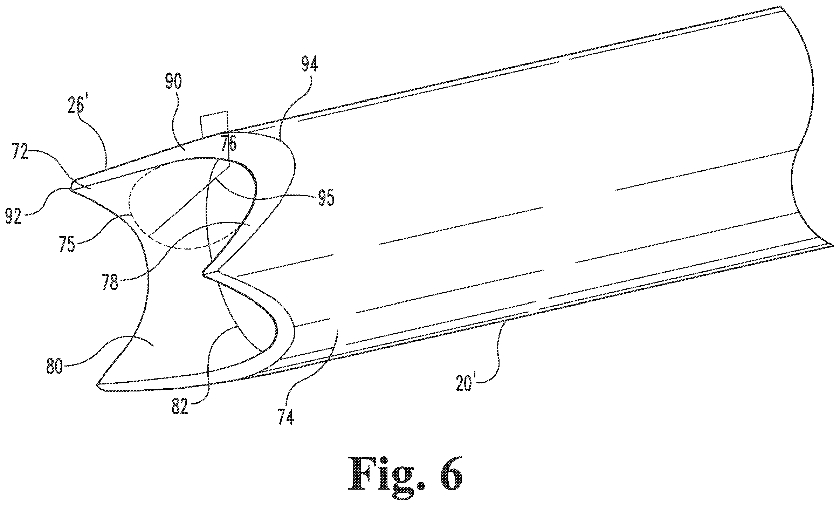

In order to address this difficulty, a full core biopsy device according to another embodiment, provides a feature for improving the ability of the outer needle to drawn in and retain a long tissue sample. According to this embodiment, as shown in **FIGS. 6-7****,** an outer needle **20'** includes an inner surface **72** and an outer surface **74.** The outer needle **20'** defines a thickness **76** between the inner surface **72** and the outer surface **74.** In one aspect, this retention feature includes a countersink or forcing cone **80** defined in the inner surface **72.** The forcing cone **80** essentially "forces" or squeezes more tissue into the outer needle as it is advanced into the tissue. It is believed that the tissue squeezed into the outer needle by the forcing cone leads to an increased friction force between the sample and the needle that holds the sample even when the sample length falls outside the desirable aspect ratio discussed above. Thus, a 16 gauge needle with the forcing cone **80** disclosed herein can obtain an intact tissue sample having a length of less than 1.0in (vs. the 1.1-1.3in. length described above). In other words, the forcing cone allows the full core biopsy device disclosed herein to obtain a viable tissue sample with a minimum stroke. Of course, longer samples may be obtained with longer strokes, if desired.

The forcing cone further leads to a sharper cutting edge **78** at the tip of the outer needle. In particular, the cutting edge of an outer needle may be formed by grinding the outer surface **74** of the needle **20'**, either to a sharp edge or to a Franseen grind as depicted in **FIG. 6****.** The forcing cone **80** may then be formed by grinding the inner surface **72** at an appropriate cone angle **γ.** The intersection between the outer and inner surface grinds thus creates a sharper cutting edge **78** than either grind alone.

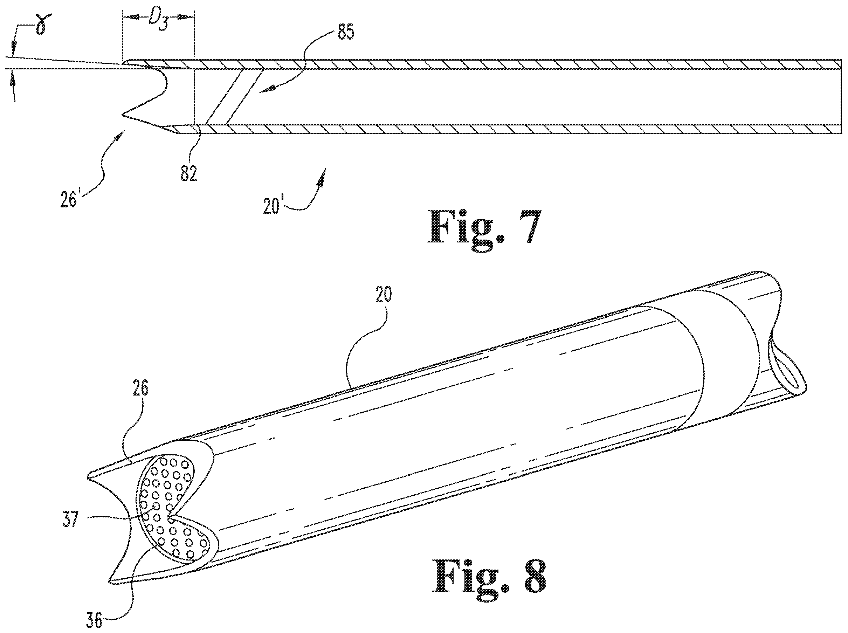

The countersink or forcing cone **80** is formed in the inner surface **72** of the outer needle **20'** and extends from the tip to an inner end **82.** The inner end **82** is located at a depth **D3** that may be, in certain embodiments, approximately twice the diameter **95** defined by the valley **75** between the prongs **92** of the tip **26'**. The forcing cone **80** is formed such that the thickness **76** of the outer needle **20'** is greater at the inner end **82** than at the tip **26'**. In other words, the thickness at the inner end **82** is equal to the wall thickness of the tubular body of the outer needle **20'** but tapers to a sharp cutting edge **78** at the tip **26'**. The forcing cone **80** is formed in the inner surface **72** at an angle **γ** as shown in **FIG. 7**. According to the invention, the angle **γ** is about 1-2° so that the countersink or forcing cone **80** forms an included angle of about 3-4°. Depending upon the wall thickness of the outer needle **20'** the angle **γ** may be as great as about 6°.

The forcing cone **80** assists in retaining the tissue within the outer needle when the device is fired and when the excised tissue is being removed. It is believed that the forcing cone tends to compress a greater volume of tissue into the outer cannula during the coring operation and that this greater volume in turn provides additional surface tension or pressure between the tissue sample and the forcing cone **80**. This increased pressure allows the tissue sample to "grip" the inner surface of the outer needle as the device is being extracted from the tissue site.
The forcing cone further leads to a sharper cutting edge **78** at the tip of the outer needle. In particular, the cutting edge of an outer needle may be formed by grinding the outer surface **74** of the needle **20'**, either to a sharp edge or to a Franseen grind as depicted in **FIG. 6** and described below. The forcing cone **80** may then be formed by grinding the inner surface **72** at an appropriate cone angle **γ**. The intersection between the outer and inner surface grinds thus creates a sharper cutting edge **78** than either grind alone, in part because the countersink or forcing cone **80** decreases the thickness **76** of the outer needle **20'**. The thickness of the cutting edge **78** may be approximately 0.0005 to 0.001 in. in certain embodiments

In another aspect, the outer needle **20'** may further include a tissue slicing feature **90** formed in the outer surface **74.** The tissue slicing feature **90** also reduces the thickness **76** of the outer needle **20'** so that the tip **26'** is the thinnest portion of the outer needle **20'**. The tissue slicing feature **90** may be, for example, a Franseen tip (as described above with reference to **FIGS. 4-5** and as shown in **FIG. 6-7**). Other suitable slicing configurations may be a Trocar tip, a Quinke tip or any other needle point feature that forms a sharp tip and edge.

In this embodiment, the thickness **76** of the outer needle **20'** varies along its length due to the introduction of the described features. The thickness **76** of the outer needle **20'** between the hub **22** and the inner end **82** of the forcing cone **80** may be approximately 0.003 or 0.004 inches. The thickness **76** of the outer needle **20'** begins to decrease by the angle **γ** at the inner end **82** of the forcing cone **80** and begins to decrease further by the angle α at the valleys **94** of the tissue slicing feature **90**. The thickness **76** at the tip **26'** may be thus reduced to approximately 0.0012 to 0.0014 inches.

The result of the embodiment described above, including both the countersink or forcing cone **80** and the tissue slicing feature **90,** is a complete and uniform core sample trapped within the end of the outer needle **20'**, without any crush artifact. The tissue slicing feature **90** of the device **20'** provides a cleaner cut with only linear motion and without rotation of the outer needle **20'**. Furthermore, the countersink or forcing cone **80** of the device **20'** provides a guiding surface to guide and support the core as it is cut away from the tissue by the cutting edge **78** of the outer needle **20'**.

The angle **γ** and the included angle of the forcing cone or countersink **80** described above is governed, at least in part, by the tissue slicing feature **90** and the wall thickness of the outer needle **20'**. As described above, in the illustrated embodiment the tissue slicing feature **90** is a Franseen tip which includes valleys or roots **94.** The countersink **80** extends from the tip **26'** and terminates proximal to the end of the Franseen valleys **94** at the depth **D3.** The depth **D3** must be sufficient to draw the tissue fully into the outer needle, which in the illustrated embodiment is twice the diameter **95** of the Franseen valley. This depth, in combination with the wall thickness of the needle, determines the maximum possible angle **γ**. A thicker wall permits a greater angle, but at the cost of either increasing the diameter of the outer needle, which increases the diameter of the wound, or decreasing the internal diameter which decreases the diameter of the tissue sample.

In certain embodiments for obtaining a clinically suitable full core biopsy, the outer needle is formed of stainless steel tubing having a gauge of between 16 and 20. A 16 gauge needle has a nominal outer diameter of 0.0650in. ±.0005 and an inner diameter of 0.0535in± .001, for a nominal wall thickness of 0.006 in. A 20 gauge needle has a nominal O.D. of .0350in. ±0005 and a nominal I.D. of 0.0295in± .001, for a nominal wall thickness of 0.003 in. A typical Franseen grind yields a valley diameter **95** of 0.04-0.05in., so the depth **D3** in the illustrated embodiment is 0.08-0.10in. These dimensions yield a possible angle **γ** in the range of 1.7-4.3° (with the shallower angle arising from a combination of the thinnest wall and the longest depth **D3**). However, the tip **26'** of the outer needle is ground to define a cutting edge, so the larger angle **γ** of 4.3° can be problematic for providing as sharp a cutting edge as possible. Likewise, while a shorter depth **D3** is possible, the result is a greater angle **γ** which may compromise the sharpness of the cutting edge. The need to maintain a sharp cutting edge also impacts the suitable angle **γ** that do not include the Franseen grind of the illustrated embodiment.

The outer needle **20'** may include other tissue retention features formed in the inner surface **72** of the outer needle in conjunction with or in lieu of the forcing cone **80**. Thus, in one feature a spiral groove **85** is formed in the inner surface **72.** The groove **85** may be formed in the inner surface **72** at a location adjacent the inner end **82** of the forcing cone **80**, as shown in **FIG. 7****.** In this embodiment, the groove **85** has a depth of 0.04 to 0.08 inches. The groove **85** is shown as commencing at the end **82** of the forcing cone, although in other embodiments the groove may overlap the forcing cone. It is believed that the groove enhances the "grip" between the outer needle and the tissue being excised, particularly when combined with the forcing cone **80**. It is contemplated that other tissue retention features may be incorporated into the inner surface **72** of the outer cannula. For instance, rather than a spiral groove, such as the groove **85,** the feature may include a series of circumferential grooves, axial grooves, striations, ridges, knurling or other features that provide an irregular surface into which the tissue may swell. However, the spiral groove may be preferred for manufacturing reasons.

In one embodiment, the outer needle **20** of the full core biopsy device **10** may include a countersink or forcing cone **80** along with a predetermined relative positioning between the inner needle **30** and the outer needle **20'**. In this embodiment, the inner needle **30** can have a length that maintains the inner needle tip **36** in a position (not shown) such that the inner needle tip **36** is situated in the outer needle **20'** between the hub **22** and the inner end **82** of the countersink or forcing cone **80**. In other words, the tip of the inner needle may be offset proximal or inboard of the inner end **82** of the forcing cone **80**. This embodiment combines the advantages provided by the relative positioning of the inner and outer needles (in the same manner as described above in reference to the inner needle **30** and outer needle **20** of **FIGS. 4-5**) with the advantages provided by the forcing cone **80** as described above. Likewise, the other retention features, such as the spiral groove **85,** may be incorporated into the full core biopsy device **20**.

The inner needle tip **36** may be closed or blocked to prevent tissue ingress into the inner needle **30**. In another approach, the inner needle **30** may be integrated into an irrigation or aspiration/vacuum system. In this approach the inner needle **30** is hollow with the proximal end coupled to an irrigation or aspiration component. In order to ensure that tissue does not enter the inner needle **30**, the tip **36** may be provided with a filter element that is configured to allow passage of fluids but not tissue.

In one embodiment, the filter element is an etched membrane filter, such as the filter **37** shown in **FIG. 8**. The filter **37** may be made of 304 stainless steel and welded to the inner diameter of the hollow inner needle **30** at or adjacent the tip **36** of the needle **30**. In one specific embodiment the membrane is about 0.002 inches thick with a series of 0.0037 inches diameter perforations distributed at a 0.0055 inches pitch around the area of the filter **37.** Other filter element configurations are contemplated, such as a wire mesh construction.

The filter element may enhance the procedure for using the biopsy device **10** described above. For instance, in some procedures it may be desirable to apply suction at the outer needle tip **26** to help draw tissue into the outer needle **20** or to hold the tissue within the outer needle **20** as the biopsy device **10** is withdrawn from the patient. The inner needle **30** may thus be coupled to a device that provides suction at the inner needle tip **36.** The filter element **37** can prevent ingress of tissue into the inner needle **30**. A full core or other biopsy device can benefit from the incorporation of the filter element **37** particularly in combination with one or more of the tissue slicing feature **90**, the tissue retention feature spiral groove **85,** the forcing cone **80** and the relative positioning of the inner needle **30** and the outer needle **20**, **20'**.

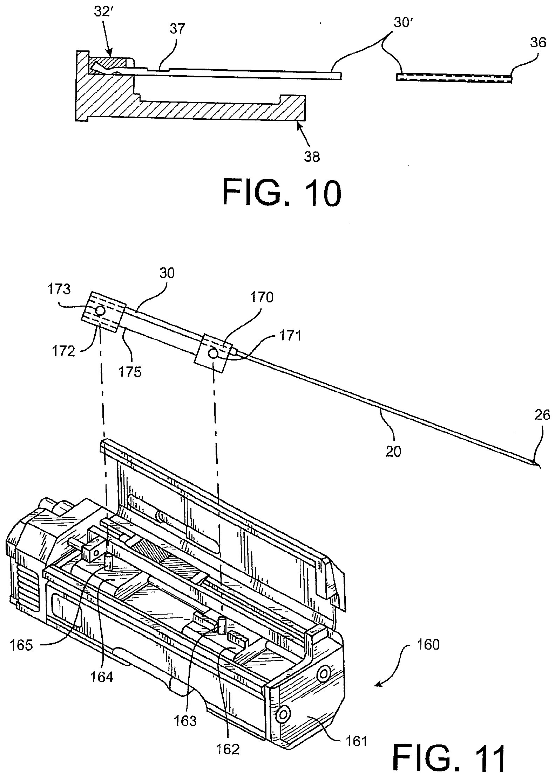

In certain embodiments, the inner needle may be modified to provide a vent feature to relieve air pressure as the tissue sample is drawn into the outer needle **20**. Thus, as shown in **FIG. 10****,** the proximal end of an inner needle **30'** is mounted in an inner needle hub **32'**. The inner needle **30'** further defines a vent opening **37** that communicates with the interior of the needle to the distal tip **36.** The vent opening **37** eliminates any pressure head behind a tissue sample as it is drawn into the outer needle which might otherwise impede the ability of the full core biopsy device to draw a complete, intact tissue sample.

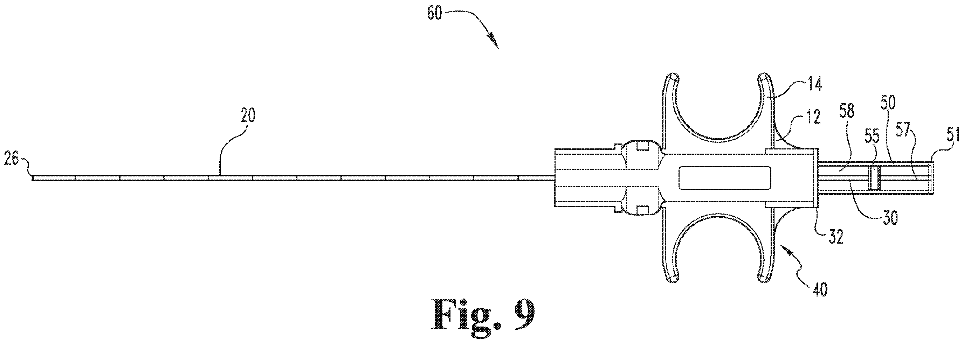

With respect to the application of suction to assist in extracting the tissue sample, it is contemplated that only a short vacuum "burst" is needed to sufficiently retain the tissue sample within the outer needle **20, 20'.** Thus, while the inner needle **30** may be connected to an external vacuum source, such an approach may not be practical or necessary for many applications of the full core biopsy device **10** disclosed herein. Consequently, a biopsy device **60** shown in **FIG. 9** may be modified to incorporate a vacuum generation feature. The device **60** may be similar to the device **10** described above, with substantially the same housing **12,** outer needle **20** and full core tip **26,** and substantially the same hub **32** supporting the inner needle **30**.

In this embodiment, the biopsy device **60** is modified to incorporate a vacuum element **50** mounted in sealed engagement with the inner needle hub **32.** The inner needle **30** has a length sufficient to extend from the outer needle tip **26** to (or slightly beyond) the open end **51** of the vacuum element **50.** A piston **55** is mounted on the proximal end of the inner needle **30** and slidably disposed within the vacuum chamber **58.** The proximal end of the inner needle **30** may be provided with a handle **57** that can be used to pull the inner needle **30** back from the tip **26** of the outer needle **20, 20'.** When the handle **57** is pulled back, the piston **55** draws a vacuum within the chamber **58,** which in turn pulls a vacuum within the inner needle **30**. The vacuum may be enhanced if the excised tissue impinges on the distal tip **36** of the inner needle **30**. The inner needle **30** may be provided with openings that communicate between the lumen of the inner needle and the vacuum chamber **58.**

In some embodiments the operation of the vacuum element **50** may be coordinated and automatic with the firing of the biopsy device **60.** If the vacuum element **50** is operated too soon before the tissue sample has been captured by the outer needle **20, 20'** little or no vacuum will be drawn within the inner needle **30**. The vacuum may thus commence when the outer needle **20, 20'** approaches the end of its cutting stroke to help draw the tissue into the outer needle **20, 20'.** The vacuum is preferably maintained until the outer needle **20**, **20'** has begun to be withdrawn from the biopsy site. In prior full core biopsy devices, when the device is being withdrawn with excised tissue, resistance in the tissue sample may tend to pull the sample back to the biopsy site and out of the biopsy device. Providing suction as the biopsy device **60** is withdrawn can resist dislodgement or retraction of the tissue sample and ensure that the sample is completely separated from the original tissue site. However, it may be preferable that the vacuum be maintained for a limited duration to avoid sucking the tissue sample into the inner needle **30** (particularly if no filter **37** is present) which may compromise the integrity of the tissue sample for subsequent histopathology. Thus, the suction may be preferably applied for a duration less than the time to fully extract the biopsy device from the tissue site.

The amount of vacuum that can be drawn by the vacuum element **50** may be limited by limiting the stroke of the piston **55** mounted to the inner needle **30**. It is contemplated that only minimal suction may be needed to ensure complete removal of the tissue sample upon withdrawal of the biopsy device **60.** It is important that integrity of the tissue sample be preserved and unaffected by the suction.

In a further embodiment, the vacuum element **50** may be configured to pull the vacuum within the outer needle **20, 20'.** In this case, the chamber **58** is in communication with the outer needle **20, 20',** while the piston **55** remains mounted on the inner needle **30**. In either embodiment, the suction is self-generated within the biopsy device **60** so no external vacuum source is necessary. This self-generation aspect insures that the amount of suction generated cannot exceed an acceptable value that might otherwise cause damage to the tissue or compromise the function of the device. Moreover, the vacuum may be generated consistently with each firing of the biopsy device **60** without the need to "reset" any components.

In some biopsy settings, it is desirable to obtain multiple tissue samples. In this case, after each firing of the biopsy device **60** the tissue sample may be dislodged by charging the outer needle **20, 20',** but the position of the inner needle **30** may be unchanged (except in some cases in which the inner needle **30** is advanced slightly to assist in dislodging the tissue sample). The inner needle **30** may be biased to a starting position, not only for obtaining the sample but for generating the optimum vacuum when a sample is taken.

In the embodiments shown herein, the full core biopsy components are utilized in a manual or partially automated device. The same components may be utilized in other biopsy devices. For example, the outer needle **20** (or **20'**) and inner needle **30** described above may be integrated into a fully automated device, such as the device **160** shown in **FIG. 11****.** In this embodiment, the outer needle **20** is mounted to a hub **170** while the inner needle is mounted to a hub **172.** The hubs are aligned by a guide pin **175** that maintains the position of the hubs for introduction into the firing device **161.** The hubs **170, 172** include a respective mounting bore **171, 173** that is used to seat the hubs on a corresponding carriage **162, 164** by way of mounting pins **163, 165.** Details of the construction and operation of one suitable automated device **160** are disclosed in U.S. Patent No. 7,309,317, the disclosure of which is incorporated herein by reference. With respect to capturing a tissue sample, the device **160** operates in a manner similar to the device **10** in that the outer needle **20, 20'** may be advanced into a tissue site to extract a tissue sample. The device **160** may use springs to propel the needles, as disclosed in the '317 Patent, or may implement other means for driving at least the outer needle into the tissue to be sampled.

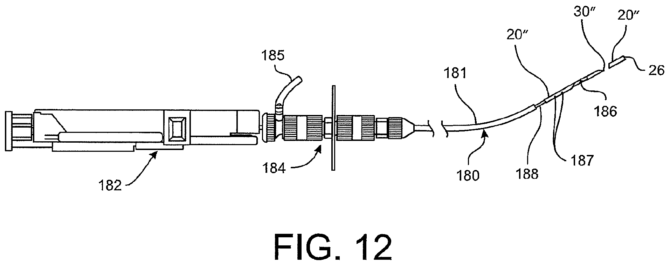

The full core components disclosed herein may also be used in guided, steerable or flexible biopsy devices. One such system is illustrated in **FIG. 12** in which the device **180** includes a flexible needle assembly **181** mounted to a firing device **182.** The device **180** may include an connector assembly **184** for incorporating an aspiration or irrigation tube **185.** The device **180** and the flexible needle assembly may be constructed and operation like the biopsy device disclosed in U.S. Patent No. 6,419,641, the disclosure of which is incorporated herein by reference. In one aspect, the flexible needle assembly includes a number of notches **186** and **188** formed in the inner needle **30"** and a number of slots **187** formed in the outer needle **20".** The notches and slots are arranged and aligned to allow the inner and outer needles to bend as the needle assembly is navigated through a body passage, such as through the jugular vein for transjugular access to the liver. The working end **26** of the outer needle **20"** is configured as shown in **FIGS. 4-5** to obtain an intact full core tissue sample. The structure and function of the inner and outer needles **20"** and **30"** are otherwise as described herein, with the primary difference being that the device **180** does not utilize the rigid needle approach of the devices **10, 60** and **160.**

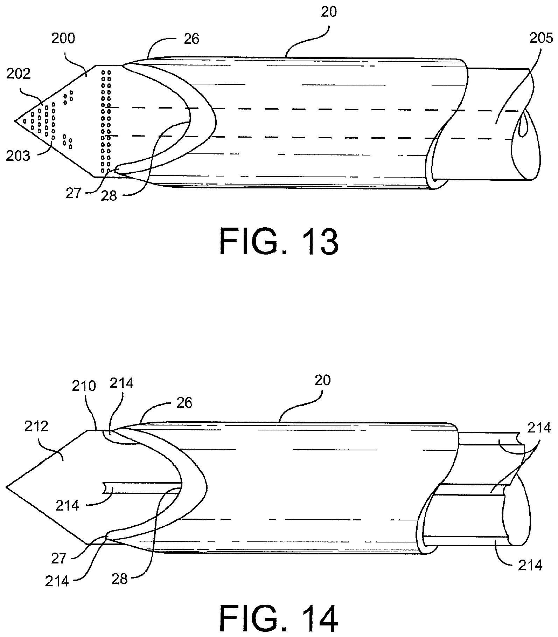

In certain procedures it is desirable to utilize a stylet to provide initial access to the biopsy site and to act as a guide wire for advancing the outer cannula. In these procedures, the stylet must be proud of the tip of the outer cannula. Thus, as shown in **FIG. 13****,** a stylet **200** extends beyond the prongs **27** of the outer cannula **20**. The stylet **200** is configured with a sharp tip **202** configured to readily penetrate tissue. In this embodiment, the tip **202** is formed of a porous material having a plurality of openings or interstices **203.** The openings communicate with an inner lumen **205** of the stylet which may be in communication with a vent or a vacuum source, as described above. The openings **203** and lumen **205** thus provide a vent that can operate in the manner described above with respect to the filter **37** shown in **FIG. 8**, namely to prevent the formation of a vacuum as the stylet is retracted relative to the outer cannula **20**.

In an alternative embodiment, a stylet **210** may be provided with a plurality of grooves **214** extending from the tip **212** along the length of the stylet. The grooves **214** may be in communication with a vent or a vacuum source, and function in the same manner as described above. It is contemplated that other features may be incorporated into the stylet that permit venting between the stylet and the outer cannula so that the extraction of an intact tissue sample is not compromised.

The foregoing detailed description of one or more embodiments of the biopsy device with an inner needle disposed within an outer needle has been presented herein by way of example and not limitation. It will be recognized that there are advantages to certain individual features and functions described herein.

## Claims

1. A biopsy device (10) comprising:
an outer needle hub (22);
an outer needle (20; 20') defining a longitudinal axis along a length thereof and coupled at a proximal end to the outer needle hub (22), the outer needle (20) including:
an outer needle tip (26) at an opposite distal end;
a tissue slicing feature (90) defined at said tip (26) configured for cutting tissue; and
an inner surface (72) including a countersink (80) formed at said tip (26), said countersink (80) tapered from a distalmost end of said outer needle tip (26);
an inner needle hub (32);
an inner needle (30) coupled at a proximal end to said inner needle hub (32), the inner needle (30) including an inner needle tip (36) at an opposite distal end, said inner needle (30) coaxially disposed within said outer needle (20), **characterized in that** said countersink (80) is tapered at approximately a one to two degree angle relative to the inner surface (72).

2. A biopsy device (10) of claim 1, wherein
said tissue slicing feature (90) includes a plurality of prongs (27) and valleys (28) between said prongs (27), said valleys (28) defining a diameter; and
said countersink (80) is tapered from said outer needle tip (26) to an inner end located at a distance from said outer needle tip (26) equal to twice said diameter.

3. The biopsy device of claim 1 or 2, wherein said tissue slicing feature is a Franseen tip (26; 90).

4. The biopsy device of claim 1, wherein said outer needle (20) includes a tissue retention groove (85) defined in said inner surface (72) between said countersink (80) and said proximal end of said outer needle (20).

5. The biopsy device of claim 1, wherein:
said tissue slicing feature (90) includes a plurality of prongs (27) and valleys (28) between said prongs (27);
said countersink (80) is tapered from said distalmost end of said outer needle tip to an inner end located between said valleys and said proximal end of said outer needle.

6. The biopsy device of claim 1, wherein said inner needle is a stylet (200) having a sharp tip (202) configured to penetrate tissue.

7. The biopsy device of claim 6, further comprising:
a mechanism coupled to said outer needle hub (22) and said inner needle hub (32) for charging and firing said outer needle (20) relative to said stylet (200) to capture a tissue sample within said outer needle tip (26),
wherein said stylet (200) and said outer needle (20) are sized so that said stylet tip (202) is initially distal a distalmost end of said outer needle tip (26) before the outer needle (30) is fired.

8. The biopsy device of claim 6, wherein said stylet (200) and said outer needle (20) are sized so that said stylet tip (202) is initially distal a distalmost end of said outer needle tip (26) before the outer needle (20) is fired.

## Patentansprüche

1. Biopsie-Vorrichtung (10), umfassend:
eine Außennadelnabe (22);
eine Außennadel (20; 20'), die entlang ihrer Länge eine Längsachse definiert und an einem proximalen Ende mit der Außennadelnabe (22) gekoppelt ist, wobei die Außennadel (20) folgendes enthält:
eine Außennadelspitze (26) an einem entgegengesetzten distalen Ende;
ein an der Spitze (26) definiertes zum Schneiden von Gewebe konfiguriertes Gewebeschneidmittel (90); und
eine innere Oberfläche (72), die eine Ansenkung (80) enthält, die an der Spitze (26) ausgebildet ist, wobei die Ansenkung (80) von einem am weitesten distal gelegenen Ende der Außennadelspitze (26) aus angeschrägt ist;
eine Innennadelnabe (32);
eine Innnennadel (30), die an einem proximalen Ende mit der Innennadelnabe (32) gekoppelt ist, wobei die Innennadel (30) eine Innennadelspitze (36) an einem entgegengesetzten distalen Ende enthält, wobei die Innennadel (30) innerhalb der Außennadel (20) koaxial angeordnet ist,
**dadurch gekennzeichnet, dass**
die Ansenkung (80) unter etwa einem Winkel von eins bis zwei Grad relativ zur inneren Oberfläche (72) angeschrägt ist.

2. Biopsie-Vorrichtung (10) nach Anspruch 1, wobei
das Gewebeschneidmittel (90) eine Vielzahl von Zacken (27) und Vertiefungen (28) zwischen den Zacken (27) enthält, wobei die Vertiefungen (28) einen Durchmesser definieren; und
die Ansenkung (80) von der Außennadelspitze (26) bis zu einem inneren Ende angeschrägt ist, das bei einem Abstand von der Außennadelspitze (26) gleich einem Zweifachen des Durchmessers angeordnet ist.

3. Biopsie-Vorrichtung nach Anspruch 1 oder 2, wobei das Gewebeschneidmittel eine Franseen-Spitze (26; 90) ist.

4. Biopsie-Vorrichtung nach Anspruch 1, wobei die Außennadel (20) eine Gewebezurückhaltenut (85) enthält, die in der inneren Oberfläche (72) zwischen der Ansenkung (80) und dem proximalen Ende der Außennadel (20) definiert ist.

5. Biopsie-Vorrichtung nach Anspruch 1, wobei:
das Gewebeschneidmittel (90) eine Vielzahl von Zacken (27) und Vertiefungen (28) zwischen den Zacken (27) enthält;
die Ansenkung (80) von dem am weitesten distal gelegenen Ende der Außennadelspitze aus bis zu einem zwischen den Vertiefungen und dem proximalen Ende der Außennadel angeordneten inneren Ende angeschrägt ist.

6. Biopsie-Vorrichtung nach Anspruch 1, wobei die Innennadel ein Stilett (200) mit einer scharfen Spitze (202) ist, die konfiguriert ist, um Gewebe zu durchdringen.

7. Biopsie-Vorrichtung nach Anspruch 6, weiterhin umfassend:
einen Mechanismus, der mit der Außennadelnabe (22) und der Innennadelnabe (32) zum Laden und Entladen bzw. Auslösen der Außennadel (20) relativ zum Stilett (200) gekoppelt ist, um eine Gewebeprobe innerhalb der Außennadelspitze (26) zu erfassen,
wobei das Stilett (200) und die Außennadel (20) so bemaßt sind, dass die Stilettspitze (202) anfangs distal zu einem am weitesten distal gelegenen Ende der Außennadelspitze (26) ist, bevor die Außennadel (30) entladen wird.

8. Biopsie-Vorrichtung nach Anspruch 6, wobei das Stilett (200) und die Außennadel (20) so bemaßt sind, dass die Stilettspitze (202) anfangs distal zu einem am weitesten distal gelegenen Ende der Außennadelspitze (26) ist, bevor die Außennadel (30) entladen bzw. ausgelöst wird.

## Revendications

1. Dispositif de biopsie (10) comprenant :
un pavillon d'aiguille externe (22),
une aiguille externe (20, 20') définissant un axe longitudinal le long de sa longueur et couplée, à une extrémité proximale, au pavillon d'aiguille externe (22), l'aiguille externe (20) incluant :
une pointe d'aiguille externe (26) à une extrémité distale opposée,
une fonction de coupe des tissus (90) définie à ladite pointe (26) configurée pour couper des tissus, et
une surface interne (72) incluant une fraise (80) formée à ladite pointe (26), ladite fraise (80) étant conique à partir de l'extrémité la plus distale de ladite pointe d'aiguille externe (26),
un pavillon d'aiguille interne (32)
une aiguille interne (30) couplée, à une extrémité proximale, audit pavillon d'aiguille interne (32), l'aiguille interne (30) incluant une pointe d'aiguille interne (36) à une extrémité distale opposée, ladite aiguille interne (30) étant disposée de manière coaxiale à l'intérieur de ladite aiguille externe (20),
**caractérisé en ce que**
ladite fraise (80) est conique, à un angle de 1 à 2 degrés, par rapport à la surface interne (72).

2. Dispositif de biopsie suivant la revendication 1, dont
ladite fonction de coupe des tissus (90) inclut une pluralité de dents (27) et de creux (28) entre lesdites dents (27), lesdits creux (28) définissant un diamètre, et
ladite fraise (80) est conique à partir de ladite pointe d'aiguille externe (26) vers une extrémité interne, située à une distance qui est égale à deux fois ledit diamètre, de ladite pointe d'aiguille externe (26).

3. Dispositif de biopsie suivant la revendication 1 ou 2, dont ladite fonction de coupe des tissus est une pointe Franseen (26, 90).

4. Dispositif de biopsie suivant la revendication 1, dont ladite aiguille externe (20) inclut une rainure destinée à retenir des tissus (85) définie dans ladite surface interne (72), entre ladite fraise (80) et ladite extrémité proximale de ladite aiguille externe (20).

5. Dispositif de biopsie suivant la revendication 1, dont :
ladite fonction de coupe des tissus (90) inclut une pluralité de dents (27) et de creux (28) entre lesdites dents (27),
ladite fraise (80) est conique à partir de l'extrémité la plus distale de ladite pointe d'aiguille externe vers une extrémité interne située entre lesdits creux et ladite extrémité proximale de ladite aiguille externe.

6. Dispositif de biopsie suivant la revendication 1, dont ladite aiguille interne est un stylet (200) ayant une pointe acérée (202) configurée pour pénétrer dans des tissus.

7. Dispositif de biopsie suivant la revendication 6, le dispositif comprenant en plus :
un mécanisme couplé audit pavillon d'aiguille externe (22) et audit pavillon d'aiguille interne (32) pour charger et expulser ladite aiguille externe (20) par rapport audit stylet (200), afin de saisir un échantillon de tissu à l'intérieur de ladite pointe d'aiguille externe (26),
ledit stylet (200) et ladite aiguille externe (20) étant dimensionnés de sorte que ladite pointe du stylet (202) est initialement distale à une extrémité la plus distale de ladite pointe d'aiguille externe (26) préalablement à l'expulsion de l'aiguille externe (30).

8. Dispositif de biopsie suivant la revendication 6, dont ledit stylet (200) et ladite aiguille externe (20) sont dimensionnés de sorte que ladite pointe de stylet (202) est initialement distale à une extrémité la plus distale de ladite pointe d'aiguille externe (26) préalablement à l'expulsion de l'aiguille externe (20).
